# EUROPÄISCHE PATENTANMELDUNG

(11) **EP 4 520 839 A2**
(43) Veröffentlichungstag der Anmeldung: **12.03.2025**
(21) Anmeldenummer: 23219887.9
(22) Anmeldetag: 22.12.2023
(51) Int. Cl.: C12P 19/02, C12P 19/36, C12N 9/90, C12N 9/04, C12N 9/02

(54) **VERFAHREN ZUR HERSTELLUNG EINER WÄSSERIGEN LÖSUNG ENTHALTEND D-PSICOSE**

(30) Priorität: 15.03.2023 EP 23162092; 15.05.2023 EP 23173451
(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Die vorliegende Erfindung betrifft ein Verfahren zur Herstellung einer wässerigen Lösung enthaltend D-Psicose, indem aus D-Fructose, welche in einer wässerigen Lösung gelöst vorliegt, durch Behandeln mit einer Epimerase *in vitro* eine erste D-Psicose gebildet wird, wonach die erste D-Psicose durch Behandeln mit einer NAD(P)H-abhängigen Oxidoreduktase *in vitro* zu Allitol reduziert und nach Deaktivierung und/oder Ultrafiltration der Epimerase zur Bildung von D-Psicose mit einer entsprechenden NAD(P)⁺-abhängigen Oxidoreduktase behandelt wird, wonach die deaktivierte Epimerase und die Oxidoreduktasen entfernt werden, wobei die NAD(P)⁺-abhängige Oxidoreduktase zur Bildung von D-Psicose aus Allitol eine Aminosäuresequenz umfasst, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet. (Figur 1)

## Beschreibung

Die vorliegende Erfindung betrifft ein Verfahren zur Herstellung einer wässerigen Lösung enthaltend D-Psicose.

### Hintergrund der Erfindung

### D-Psicose

Das Monosaccharid D-Psicose, auch bekannt als D-Allulose, ist eine in der Natur selten vorkommende Ketohexose (Zhang et al., 2016). Sie konnte unter anderem in den Blättern von Rosmarinweiden (*Itea* sp.) nachgewiesen werden (Hough & Stacey, 1966), findet sich aber auch in verarbeiteten Lebensmitteln wie Konfekt und Gewürzsaucen, wo sie unter Einwirkung von Hitze aus D-Fructose, ihrem C₃-Epimer, entsteht (Oshima et al., 2006).

D-Psicose ist aufgrund ihres süßen Geschmacks für die Lebensmittelindustrie interessant. Im Vergleich zur Saccharose weist D-Psicose eine relative Süßkraft von 70% auf, besitzt aber einen niedrigen Energiegehalt (0,2 kcal/g), was einer Kalorien-Reduktion von etwa 95% (bezogen auf Saccharose) gleichkommt (Jiang et al., 2020).

In den USA ist D-Psicose von der US-amerikanischen Lebensmittelaufsicht Food and Drug Administration (FDA) als *Generally Recognized as Safe* (GRAS)-Süßungsmittel anerkannt, in der EU jedoch noch nicht zugelassen (Ahmed et al., 2022).

Darüber hinaus hat D-Psicose auch positive Effekte auf den Lipid-Stoffwechsel sowie den Kohlenhydrat-Stoffwechsel (z.B. antidiabetisch) und wirkt entzündungshemmend und antioxidativ (Zhang et al., 2016; Jiang et al., 2020; Chen et al., 2022).

Aufgrund des geringen natürlichen Vorkommens wird D-Psicose weitgehend synthetisch (chemisch oder biotechnologisch) hergestellt.

Die Epimerisierung von D-Fructose zu D-Psicose kann durch Kochen in Pyridin unter Rückfluss mit anschließender Entfernung der anderen Hexosen durch Hefe-Fermentation durchgeführt werden, wobei allerdings nur 6,8 % der theoretischen Ausbeute an D-Psicose erreicht werden (Doner, 1979). Eine andere Methode beinhaltet die Epimerisierung von D-Fructose mit Molybdat-Ionen als Katalysator, wobei nur 0,5% der D-Fructose zu D-Psicose umgesetzt werden (Bilik & Tihlärik, 1974).

Die ineffizienten chemischen Syntheserouten wurden mittlerweile durch effizientere biotechnologische Verfahren ersetzt. Izumori et al. beschrieben 1993 eine Ketose-3-Epimerase aus *Pseudomonas cichorii* ST-24 zur Herstellung von D-Psicose aus D-Fructose (Izumori et al., 1993), welche auch patentiert wurde (EP 0592202 B1). Ketose-3-Epimerasen lassen sich je nach Substratspezifität in drei Gruppen einteilen: 1) D-Tagatose-3-Epimerase (DTE), 2) D-Psicose-3-Epimerase (DPE) oder D-Allulose-3-Epimerase (DAE) und 3) L-Ribulose-3-Epimerase (LRE) (Zhang et al., 2016; Jiang et al., 2020).

Die Umsetzung von D-Fructose zu D-Psicose mittels Ketose-3-Epimerasen läuft jedoch nicht vollständig ab, vielmehr bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose). Viele der Epimerasen benötigen außerdem ein zweiwertiges Metallion wie Mn²⁺ oder Co²⁺ (toxisch) als Kofaktor (Zhang et al., 2016; Jiang et al., 2020).

Das Gleichgewicht bei der Epimerisierung kann nicht nur durch Temperatur oder pH-Wert beeinflusst werden, sondern auch durch die Zugabe von (toxischem) Borat. Bedingt durch die bevorzugte Ausbildung eines D-Psicose-Borat-Komplexes wird das Gleichgewicht zur D-Psicose hin verschoben (Kim et al., 2008; Lim et al., 2009). EP 3643786 A2 und US 11028420 B2 beschreiben die chromatographische Auftrennung des D-Psicose-Borat-Komplexes mittels Simulated Moving Bed-(SMB)- Chromatographie. EP 3395952 B1 und US 10550414 B2 legen offen, dass die Konversion bei der Epimerisierung mittels DPE bei Zusatz von Natriumaluminat auf bis zu 67 % und bei Kaliumiodat auf bis zu 52% erhöht werden kann (Vergleich: 25% ohne Zusatz von Aluminat oder Iodat).

Zhu et al. (2020) präsentierten ein System bestehend aus zwei Enzymen (exo-Inulase aus *Bacillus velezenis* und DAE aus *Ruminococcus* sp.), mit dem Inulin aus *Helianthus tuberosus* L. (Topinambur) in ein Sirup bestehend aus D-Glucose, D-Fructose und D-Psicose (1:3:1) umgewandelt werden kann. Li et al. (2021a) verwendeten ein System bestehend aus Invertase, D-Glucose-Isomerase und immobilisierter DAE aus *Pirellula* sp. SH-Sr6A, um Saccharose, D-Glucose und D-Fructose (aus Fruchtsäften) in D-Psicose umzuwandeln. Es konnten 16 - 19% D-Psicose (bezogen auf den Gesamtgehalt der Kohlenhydrate) in den Säften angereichert werden.

Juneja et al. (2019) analysierten in einer Studie die technoökonomischen Aspekte eines modifizierten Corn-Dry-Grind-Prozesses, bei dem aus gemahlenem Mais zusätzlich zu Ethanol durch Einsatz eines modifizierten Hefestammes auch D-Psicose gebildet wird (durch Expression einer DPE). Die Autoren errechneten, dass aus einer Tonne Mais 390,4 I Ethanol und 75,3 kg D-Psicose gewonnen werden können und dass der minimale Verkaufspreis für die nach dem beschriebenen Verfahren erzeugte D-Psicose bei 1,29 US$/kg liegt (im Vergleich zum Marktpreis von 10 - 20 US$/kg im Jahr 2018). WO 2020/057560 A1 sowie WO 2020/057561 A1 beschreiben die Herstellung von D-Psicose aus Stärke mittels Saccharifizierung, enzymatischer Isomerisierung und Epimerisierung.

Patel et al. (2018) verwendeten eine auf magnetischen Eisenoxid-Nanopartikeln immobilisierte Smt3-DPE (Fusionsprotein) zur Herstellung von D-Psicose aus D-Fructose aus Obst-Trester-Waschlösungen. Die immobilisierte Epimerase konnte 20% der D-Fructose umsetzen und wurde nach Beendigung der Reaktion mit einem Magneten abgetrennt.

Yang et al. (2018) transferierten das DPE-Gen aus *Agrobacterium tumefaciens* in das thermotolerante Bakterium *Kluyveromyces marxianus.* So konnten in 12 h bei 55 °C aus 750 g/L D-Fructose 190 g/L D-Psicose hergestellt werden und die verbliebene D-Fructose wurde von *K. marxianus* zu Ethanol fermentiert. Dedania et al. (2020) immobilisierten DPE aus *A. tumefaciens* auf Titandioxid-Nanopartikeln und konnten damit 36% der D-Fructose zu D-Psicose umsetzen. Darüber hinaus konnte das immobilisierte Enzym bis zu neunmal wiederverwendet werden.

Die bei der Epimerisierung von D-Fructose entstehenden D-Fructose/D-Psicose-Mischungen können entweder durch chromatographische Verfahren oder durch die "biologische Methode" (Fermentation der überschüssigen D-Fructose zu z.B. Ethanol) getrennt werden (Jiang et al., 2020). In US 2021/0189441 A1 wird die Trennung einer D-Fructose/D-Psicose-Mischung beschrieben, wobei die D-Fructose durch einen probiotischen Mikroorganismus (*Lactobacillus* oder *Saccharomyces*) in L-Milchsäure umgewandelt wird. EP 3423460 B1 beschreibt ein Verfahren zur Aufreinigung einer D-Fructose/D-Psicose-Mischung und zur Gewinnung hochreiner D-Psicose. EP 3553069 A1 sowie Van Duc Long et al. (2009) legen eine Methode zur Trennung von D-Psicose und D-Fructose basierend auf SMB-Chromatographie offen.

Die Herstellung von D-Psicose über die Epimerase-Route hat allerdings folgende Nachteile: 1) Lage des Gleichgewichts aufseiten der D-Fructose, 2) Zusatz von (zum Teil toxischen) Metallionen als Kofaktoren für viele Epimerasen, 3) niedrige Aktivität und Langzeitstabilität der Epimerasen und 4) aufwendige Trennung des Produktgemisches.

Eine Möglichkeit zur Umgehung der thermodynamisch ungünstigen Epimerisierung sind Enzym-Kaskaden mit phosphorylierten Intermediaten. Der letzte Schritt, die Dephosphorylierung, ist irreversibel und treibt somit die Kaskade an (Li et al., 2021b).

Eine Kaskade, die in nahezu identischer Form sowohl von Li et al. (2021b) als auch von US 11168342 B2 und US 10907182 B2 beschrieben wird, weist D-Glucose-1-phosphat (G1P) als zentrales Intermediat auf. G1P wird mittels Phosphoglucomutase zuerst zu D-Glucose-6-phosphat (G6P) und dann weiter mittels Glucose-6-phosphat-Isomerase zu D-Frucose-6-phosphat (F6P) umgewandelt. F6P wird danach mittels D-Allulose-6-phosphat-Epimerase zu D-Psicose-6-phosphat epimerisiert, welches anschließend mit D-Allulose-6-phosphat-Phosphatase zur D-Psicose dephosphoryliert wird.

G1P kann beispielsweise durch die Einwirkung von Phosphorylasen auf z.B. Maltose und Amylodextrine (erhalten durch die Hydrolyse von Stärke), Cellodextrine (erhalten durch die Hydrolyse von Cellulose) oder Saccharose unter Verbrauch von Phosphat direkt hergestellt werden. Da die endständigen Zuckermonomere der Oligo- und Polysaccharide von den entsprechenden Phosphorylasen nicht phosphoryliert werden können, muss auf die Polyphosphat-Glucokinase (D-Glucose → G6P) oder Polyphosphat-Fructokinase (D-Fructose --> F6P) zurückgegriffen werden, wobei noch zusätzlich Polyphosphate als Phosphat-Quelle zugesetzt werden müssen, um die Ausbeuten zu steigern (US 11168342 B2; US 10907182 B2). Als Substrat für die Kaskade von Li et al. (2021b) dient Stärke, welche zu D-Psicose mit Ausbeuten von 79% (bei 50 g/L Substrat-Konzentration; Reaktionszeit 24 h) umgewandelt wird.

Wang et al. entwickelten zur Herstellung von D-Psicose ebenfalls eine enzymatische Kaskade ausgehend von Stärke, welche allerdings über mehrere Schritte zu Glyceraldehyd-3-phosphat und Dihydroxyacetonphosphat umgesetzt wird. Dihydroxyacetonphosphat wird mit D-Glyceraldehyd unter Einwirkung von L-Fuculose-1-phosphat-Aldolase (FucA) zu D-Psicose-1-phosphat umgewandelt, welches mittels Phosphatase zu D-Psicose dephosphoryliert wird (95 % Ausbeute bei einem Titer von 15,2 mM). Glyceraldehyd-3-phosphat wird weiter zu 2-Deoxy-D-ribose umgesetzt (Wang et al., 2020).

Auch eine Enzymkaskade ausgehend von Glycerin ist in der Literatur beschrieben. Dieses wird zu Dihydroxyacetonphosphat (Phosphorylierung von Glycerin mit einer sauren Phosphatase und nachfolgende Oxidation mit einer Glycerinphosphat-Oxidase) und D-Glyceraldehyd (Oxidation von Glycerin mit Alditol-Oxidase) umgesetzt, welche wiederum als Substrat für eine Aldolase (wie z.B. FucA) dienen. Nach Abspaltung der Phosphatgruppe wird eine Mischung aus D-Sorbose und D-Psicose erhalten, die chromatographisch getrennt werden kann (Li et al., 2020). WO 2016/201110 A1 wiederum beschreibt eine Methode zur Herstellung von D-Psicose aus Dihydroxyaceton und D-Glyceraldehyd mittels Fructose-6-phosphat-Aldolase und DTE (Zwischenprodukt D-Fructose).

Xiao et al. koppelten die Epimerisierung von D-Fructose mit der Umwandlung der D-Psicose zu D-Psicose-1-phosphat mittels L-Rhamnulose-Kinase (unter Verbrauch von Adenosintriphosphat (ATP)), um das Gleichgewicht der Epimerisierung zu verschieben. Durch nachfolgende Abspaltung der Phosphat-Gruppe durch eine saure Phosphatase wird D-Psicose erhalten (99% Umsatz von 20 mM D-Fructose). ATP muss allerdings unter Zusatz von Polyphosphat mit einer Polyphosphat-Kinase regeneriert werden (Xiao et al., 2019).

Ein großer Nachteil der Routen mit phosphorylierten Intermediaten ist der Einsatz von teuren, energiereichen Phosphat-Verbindungen wie Polyphosphat oder ATP in stöchiometrischen Mengen zur Einführung der Phosphat-Gruppen. Durch die Verwendung von Phosphorylasen kann dieses Problem zum Teil umgangen werden, jedoch können endständige Monosaccharide nicht ohne Zuhilfenahme von energiereichen Phosphat-Verbindungen phosphoryliert werden. Außerdem müssen die verbliebenen Phosphat-Verbindungen und Phosphat-Ionen nach Beendigung der Reaktion entfernt werden.

Auch Fermentationsprozesse zur Herstellung von D-Psicose sind bekannt. Zhang et al. (2021) präsentierten einen Fermentationsprozess basierend auf der Kokultivierung von engineerten *Bacillus subtilis* und *Escherichia coli* zur gemeinsamen Produktion von D-Psicose (Titer 11,7 g/l; Umsatz: 69,5%) und dem Enzym Lipase. In US 2017/0298400 A1 wird die Expression von DPE (z.B. aus *Agrobacterium tumefaciens)* in verschiedenen Mikroorganismen beschrieben. EP 3088515 B1 und US 9701953 B2 beschreiben einen D-Psicose produzierenden *Ensifer adhaerens-Stamm.* In EP 2470668 B1 wird die Immobilisierung eines GRAS-Mikroorganismus (*Corynebacterium glutamicum* KCCM 11046) mit exprimierter DPE auf einem Natriumalginat-Träger offengelegt.

### Allitol als Intermediat

Die unvorteilhafte Lage des Gleichgewichts der Epimerisierung von D-Fructose zu D-Psicose kann auch durch nachgeschaltete Redoxreaktionen günstig beeinflusst werden. Durch Kombination einer DTE mit einer Ribitol-Dehydrogenase (RDH; EC 1.1.1.56) und Formiat-Dehydrogenase (FDH; als Regenerationsenzym für den Kofaktor Nicotinamidadenindinukleotid NADH) kann D-Fructose *in vitro* zum Zuckeralkohol Allitol umgewandelt werden (Takeshita et al., 2000).

Durch einen Oxidationsschritt kann Allitol im Anschluss wieder zu D-Psicose umgesetzt werden. Dies kann beispielsweise mikrobiell mit *Enterobacter aerogenes* IK7 (vollständige Oxidation von 100 g/l Allitol in 24 h) oder *Bacillus pallidus* Y25 (48% Umsatz von 50 g/l Allitol in 48 h) bewerkstelligt werden (Gullapalli et al., 2007; Poonperm et al., 2007).

Der achirale Zuckeralkohol Allitol bildet aufgrund seiner Symmetrie eine Schnittstelle zwischen den D-und L-Hexosen in der sogenannten Izumoring-Strategie zur Bioproduktion von seltenen Zuckern (Izumori, 2006; Hassanin et al., 2017). Somit kann er auch als Vorstufe zur Herstellung von weiteren seltenen Monosacchariden dienen.

Ebenfalls durch mikrobielle Oxidation kann Allitol zu L-Psicose umgewandelt werden - mit *Gluconobacter frateurii* IFO 3254 können 100 g/l Allitol zu 98% zu L-Psicose oxidiert werden (Takeshita et al., 1996). Die Oxidation durch denselben Organismus ist auch in JP4761424B2 sowie JP3711296B2 beschrieben.

Wang et al. (2023) beschreiben ein System bestehend aus zwei E. coli-Ganzzell-Biokatalysatoren zur Umwandlung von D-Fructose (90 g/l) zu D-Psicose mit einem Umsatz von 90%. Im ersten Schritt (Umsetzung von D-Fructose zu Allitol) wurden *E. coli*-Zellen, welche eine DPE aus *Clostridiales,* eine RDH aus *Providencia alcalifaciens,* eine FDH aus *Starkeya* sowie eine weitere DPE aus *Rhizobium straminoryzae* beinhalteten, verwendet. Auf diese Weise wurde D-Fructose (500 mM = 90 g/l) innerhalb von 12 h bei 37 °C und pH 6 unter Einsatz von 1000 mM Natrium-Formiat (zwei Äquivalente bezogen auf D-Fructose) zu 452 mM Allitol umwandelt (Umsatz 90,4%). Als Nebenprodukt entstand ca. 30 mM D-Sorbitol (das Reduktionsprodukt der D-Fructose). Die Zellen wurden durch Zentrifugation abgetrennt und ausgetretene Proteine im Allitol-haltigen Überstand wurden durch Hitzeeinwirkung deaktiviert. Danach wurden *E. coli*-Zellen, welche eine RDH aus *Rubrivivax* sp. und eine NADH-Oxidase aus *Streptococcus pyogenes* beinhalteten, zur Allitol-Lösung zugesetzt. Allitol (452 mM) wurde innerhalb von 24 h bei pH 7 zu D-Psicose (450 mM) und das Nebenprodukt D-Sorbitol wurde wieder zu D-Fructose oxidiert. Der Gesamtumsatz des Verfahrens betrug 90%.

Die Verwendung einer FDH zur Kofaktor-Regeneration weist einige Nachteile auf: 1. Bildung von klimaschädlichem CO₂ als Oxidationsprodukt von Formiat, 2. Verschiebung des pH-Werts der Reaktion in den basischen Bereich (Neuhauser et al., 1998; Kratzer et al., 2015), 3. Produktion großer Mengen an Abfall bestehend aus nicht umgesetztem Natrium-Formiat und Natriumsulfat (bei Verwendung von Schwefelsäure zur pH-Kontrolle) sowie 4. die geringe spezifische Aktivität von Formiat-Dehydrogenasen (Boldt & Ansorge-Schumacher, 2020; Tishkov & Popov, 2004).

Hier setzt nun die Aufgabe der vorliegenden Erfindung an. Die Erfindung möchte ein effizientes und umweltfreundliches Verfahren zur Herstellung von wässerigen Lösungen enthaltend D-Psicose mit hohem Umsatz zur Verfügung zu stellen, welches insbesondere im Eintopf-Verfahren durchgeführt werden kann.

### Detaillierte Beschreibung der Erfindung

Die Aufgabe wird erfindungsgemäß gelöst, indem aus D-Fructose, welche in einer wässerigen Lösung gelöst vorliegt, durch Behandeln mit einer Epimerase *in vitro* eine erste D-Psicose gebildet wird, wonach die erste D-Psicose durch Behandeln mit einer NAD(P)H-abhängigen Oxidoreduktase *in vitro* zu Allitol reduziert und nach Deaktivierung und/oder Ultrafiltration der Epimerase zur Bildung von D-Psicose mit einer entsprechenden NAD(P)⁺-abhängigen Oxidoreduktase behandelt wird, wonach die deaktivierte Epimerase und die Oxidoreduktasen entfernt werden, wobei die NAD(P)⁺-abhängige Oxidoreduktase zur Bildung von D-Psicose aus Allitol eine Aminosäuresequenz umfasst, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet.

Das erfindungsgemäße Verfahren ist in der beiliegenden Figur schematisch dargestellt.

Es hat sich überaschenderweise gezeigt, dass die erfindungsgemäß gesetzten Ziele erreicht werden können, wenn die Umsetzung nicht fermentativ durchgeführt wird, sondern die Enzyme als solche in der wässerigen Lösung enthalten sind, also *in vitro* gearbeitet wird.

Eine bevorzugte Variante des erfindungsgemäßen Verfahrens besteht darin, dass der durch die Reduktion von D-Psicose zu Allitol entstandene oxidierte Kofaktor NAD(P)⁺ mittels einer Alkoholdehydrogenase (ADH) und einem sekundären Alkohol unter Bildung eines Ketons reduziert wird, wobei der sekundäre Alkohol bevorzugt 2-Propanol (Isopropanol) ist. 2-Propanol ist ein sehr günstiger Wasserstoff-Donor zur Regeneration von NAD(P)H und das Oxidationsprodukt Aceton ist aufgrund seiner Flüchtigkeit leicht abtrennbar (Xu et al., 2021). Aus dem Abgasstrom gewonnenes Aceton kann heterogen-katalytisch wieder zu 2-Propanol hydriert werden (Al-Rabiah et al., 2022), entweder in der Gasphase oder in Lösung, vorliegend als 2-Propanol/Aceton/Wasser-Mischungen oder als Aceton/Wasser-Mischungen, wobei in Zukunft vermehrt auf Wasserstoff aus nachhaltigen Quellen ("grüner Wasserstoff") gesetzt werden könnte. Die Verwendung eines solchen Systems erlaubt das Recycling von 2-Propanol ohne die Emission von klimaschädlichem CO₂ und vermeidet darüber hinaus große Abfallmengen (wie nicht umgesetztes Natrium-Formiat beim FDH-Regenerationssystem).

Die Regeneration des Kofaktors mittels ADH ist z.B. aus der EP 2812439 B1 vorbekannt oder von Xu et al. (2021) beschrieben.

In einer weiteren bevorzugten Ausführungsform der vorliegenden Erfindung wird der durch die Reduktion entstandene oxidierte Kofaktor NAD(P)⁺ mittels einer Glucose-Dehydrogenase und D-Glucose unter Bildung von D-Gluconat reduziert. Der Einsatz einer Glucose-Dehydrogenase zur Regeneration von NAD(P)H ist besonders vorteilhaft, da im Zuge der Reduktion von NAD(P)⁺ aus D-Glucose D-Gluconat gebildet wird, welches aus der Reaktionsmischung gewonnen und in verschiedensten Bereichen eingesetzt werden kann (z.B. in Metallbeizmittel, in Medikamenten und als Stabilisator in Lebensmitteln usw.). Zudem ermöglicht die Verwendung von Glucose-Dehydrogenase ein Gemisch umfassend D-Fructose und D-Glucose als Substrat zur Herstellung von Allitol bzw. D-Psicose einzusetzen, ohne dass zusätzliche D-Glucose der Reaktionsmischung zugesetzt wird und ohne dass D-Glucose zuvor in D-Fructose isomerisiert wird. Gemische aus D-Fructose und D-Glucose können beispielsweise durch Hydrolyse von Saccharose hergestellt werden. Besonders bevorzugt wird eine Glucose-Dehydrogenase eingesetzt, die von *Priestia megaterium* stammt und die eine Aminosäuresequenz umfasst, die unter der NCBI-Zugangsnummer MDQ0804260.1 erhältlich ist.

Eine weitere bevorzugte Variante des erfindungsgemäßen Verfahrens ist dadurch gekennzeichnet, dass es als Eintopf-Reaktion ohne Isolierung von Zwischenprodukten durchgeführt wird.

Im erfindungsgemäßen Verfahren werden die Enzyme vorzugsweise als Lysat der entsprechenden, sie bildenden Zellen eingesetzt. Im Gegensatz zu dem von Wang et al. (2023) beschriebenen Verfahren, das auf *E. coli*-Ganzzellen-Biokatalysatoren mit koexprimierten rekombinanten Enzymen beruht, werden die Enzyme einzeln in geeigneten *E. coli*-Produktionsstämmen exprimiert. Das erlaubt eine Optimierung der Enzymverhältnisse zueinander und dieses ist somit unabhängig vom Expressionslevel im Gesamtkonstrukt im Vergleich zu Wang et al. (2023).

Vor der Ausführung des letzten Schritts (Oxidation) werden die Enzyme (Epimerase und Reduktase und/oder Dehydrogenase) des ersten Schritts (D-Fructose --> Allitol) durch Hitzeeinwirkung deaktiviert und/oder durch Ultrafiltration entfernt, um die Bildung von Nebenprodukten durch die Enzyme zu verhindern. Besonders im Fall von D-Psicose würde ohne die entsprechende Behandlung ein Großteil der durch die Oxidation entstandenen D-Psicose von der Epimerase wieder zu D-Fructose umgewandelt werden.

Die Regeneration der Nicotinamid-basierenden Kofaktoren (NAD oder NADP) erfolgt im Falle der Reduktion der D-Psicose zu Allitol mit einer NAD(P)-abhängigen Alkoholdehydrogenase oder Glucose-Dehydrogenase und im Falle der Oxidationsreaktionen (zweiter Schritt) mit einer H₂O-bildenden NAD(P)H-Oxidase.

Die besonders bevorzugte Konzentration von D-Fructose beträgt 50 - 250 g/l.

Der besonders bevorzugte Temperaturbereich für den ersten Schritt (Epimerisierung und Reduktion) liegt zwischen 25 und 45 °C, für den zweiten Schritt (Oxidation) zwischen 20 und 30 °C.

Der besonders bevorzugte pH-Bereich beider Schritte liegt zwischen 7 und 8,5.

Bei einer weiteren, bevorzugten Variante des erfindungsgemäßen Verfahrens liegen die Enzyme in einer Suspension und/oder im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und als Paste verwendet oder in einem geeigneten Puffersystem suspendiert. Im Gegensatz zu fermentativen Verfahren, bei denen auch mit ganzen Zellen gearbeitet wird, können die *resting cells* aufgrund des nicht Vorhandensein von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

In einer weiteren Variante können die Enzyme in Pulverform, in lyophilisierter oder sprühgetrockneter Form vorliegen.

In einer besonders bevorzugten Ausführung des Verfahrens werden für die Konversion des Startmaterials nur Enzyme aus den Enzymgruppen Epimerasen und Oxidoreduktasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Die zur Reduktion von D-Psicose und zur Oxidation von Allitol verwendeten Enzyme stammen aus der Gruppe der Oxidoreduktasen (siehe Tabelle 1 für Details).

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die NAD(P)-abhängige Alkoholdehydrogenase zur Kofaktor-Regeneration umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 18 von mindestens 80% aufweist,
ii) einer Aminosäuresequenz, die durch eine Nukleinsäure kodiert wird, die eine Identität zu SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 17 bindet.

Besonders geeignet zur Kofaktor-Regeneration im Allgemeinen ist eine Alkoholdehydrogenase, deren Aminosäuresequenz mindestens 80% ident zu SEQ ID Nr. 18 ist bzw. welche durch eine Nukleinsäure kodiert wird, die eine Identität zu SEQ ID Nr. 17 von mindestens 80% aufweist bzw. unter stringenten Bedingungen an ein Nukleinsäuremolekül mit der Nukleinsäuresequenz SEQ ID Nr. 17 bindet.
SEQ ID Nr. 17: 
ATGAAAGCTGCAGTTGTGGAACAATTTAAAAAGCCGTTACAAGTGAAAGAAGTGGAAAAACCTAAGAT
CTCATACGGGGAAGTATTAGTGCGCATCAAAGCGTGTGGGGTATGCCATACAGACTTGCATGCCGCAC
ATGGCGACTGGCCTGTAAAGCCTAAACTGCCTCTCATTCCTGGCCATGAAGGCGTCGGTGTAATTGAAG
AAGTAGGTCCTGGGGTAACACATTTAAAAGTTGGAGATCGCGTAGGTATCCCTTGGCTTTATTCGGCGT
GCGGTCATTGTGACTATTGCTTAAGCGGACAAGAAACATTATGCGAACGTCAACAAAACGCTGGCTATT
CCGTCGATGGTGGTTATGCTGAATATTGCCGTGCTGCAGCCGATTATGTCGTAAAAATTCCTGATAACTT
ATCGTTTGAAGAAGCCGCTCCAATCTTTTGCGCTGGTGTAACAACATATAAAGCGCTCAAAGTAACAGG
CGCAAAACCAGGTGAATGGGTAGCCATTTACGGTATCGGCGGGCTTGGACATGTCGCAGTCCAATACG
CAAAGGCGATGGGGTTAAACGTCGTTGCTGTCGATTTAGGTGATGAAAAACTTGAGCTTGCTAAACAA
CTTGGTGCAGATCTTGTCGTCAATCCGAAACATGATGATGCAGCACAATGGATAAAAGAAAAAGTGGG
CGGTGTGCATGCGACTGTCGTCACAGCTGTTTCAAAAGCCGCGTTCGAATCAGCCTACAAATCCATTCG
TCGCGGTGGTGCTTGCGTACTCGTCGGATTACCGCCGGAAGAAATACCTATTCCAATTTTCGATACAGT
ATTAAATGGAGTAAAAATTATTGGTTCTATCGTTGGTACGCGCAAAGACTTACAAGAGGCACTTCAATT
TGCAGCAGAAGGAAAAGTAAAAACAATTGTCGAAGTGCAACCGCTTGAAAACATTAACGACGTATTCG
ATCGTATGTTAAAAGGGCAAATTAACGGCCGCGTCGTGTTAAAAGTAGATTAA
SEQ ID Nr. 18:

MKAAVVEQFKKPLQVKEVEKPKISYGEVLVRIKACGVCHTDLHAAHGDWPVKPKLPLIPGHEGVGVIEEV
GPGVTHLKVGDRVGIPWLYSACGHCDYCLSGQETLCERQQNAGYSVDGGYAEYCRAAADYVVKIPDNLS
FEEAAPIFCAGVTTYKALKVTGAKPGEWVAIYGIGGLGHVAVQYAKAMGLNVVAVDLGDEKLELAKQLG

ADLVVNPKHDDAAQWIKEKVGGVHATVVTAVSKAAFESAYKSIRRGGACVLVGLPPEEIPIPIFDTVLNGV
KIIGSIVGTRKDLQEALQFAAEGKVKTIVEVQPLENINDVFDRMLKGQINGRVVLKVD

Die hier angeführte Alkoholdehydrogenase zur Kofaktor-Regeneration umfasst vorzugsweise eine Aminosäuresequenz, die eine Identität zu SEQ ID Nr. 18 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Alkoholdehydrogenase zur Kofaktor-Regeneration die Aminosäuresequenz SEQ ID Nr. 18 oder besteht aus dieser.

Alternativ umfasst die Alkoholdehydrogenase zur Kofaktor-Regeneration vorzugsweise eine Aminosäuresequenz, die durch eine Nukleinsäure kodiert wird, die eine Identität zu SEQ ID Nr. 18 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Alkoholdehydrogenase zur Kofaktor-Regeneration kodiert, die Nukleinsäuresequenz SEQ ID Nr. 17 oder besteht aus dieser.

Ein weiterer Aspekt der vorliegenden Erfindung betrifft die Verwendung einer Alkoholdehydrogenase zur Kofaktor-Regeneration, wobei die Alkoholdehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 18 von mindestens 80% aufweist,
ii) einer Aminosäuresequenz, die durch eine Nukleinsäure kodiert wird, die eine Identität zu SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 17 bindet.

Die hier offenbarten Alkoholdehydrogenasen können zur Regeneration von NAD(P)⁺ bzw. NAD(P)H, d.h. zur Reduktion von NAD(P)⁺ bzw. zur Oxidation von NAD(P)H, in verschiedensten enzymatischen Reaktionen eingesetzt werden. Besonders bevorzugt ist der Einsatz der erfindungsgemäßen Alkoholdehydrogenasen bei der Kofaktor-Regeneration von NAD(P)⁺, welches bei der Reduktion von D-Psicose zu Allitol mittels einer NAD(P)H-abhängigen Oxidoreduktase entsteht.

Die zur Kofaktor-Regeneration eingesetzte Glucose-Dehydrogenase (GDH) kann aus einer der Gruppen EC 1.1.1.47 (Glucose-1-dehydrogenase), EC 1.1.1.118 (Glucose-1-dehydrogenase (NAD⁺)), EC 1.1.1.119 (Glucose-1-dehydrogenase (NADP⁺)) oder EC 1.1.1.360 (Glucose/Galactose-1-dehydrogenase) stammen.

Die NAD(P)-abhängige Glucose-Dehydrogenase zur Kofaktor-Regeneration umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 20 von mindestens 80% aufweist,
ii) einer Aminosäuresequenz, die durch eine Nukleinsäure kodiert wird, die eine Identität zu SEQ ID Nr. 19 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 19 bindet.

Besonders geeignet zur Kofaktor-Regeneration im Allgemeinen ist eine Glucose-Dehydrogenase, deren Aminosäuresequenz mindestens 80% ident zu SEQ ID Nr. 20 ist bzw. welche durch eine Nukleinsäure kodiert wird, die eine Identität zu SEQ ID Nr. 19 von mindestens 80% aufweist bzw. unter stringenten Bedingungen an ein Nukleinsäuremolekül mit der Nukleinsäuresequenz SEQ ID Nr. 19 bindet.
SEQ ID Nr. 19: 
ATGTATACAGATTTAAAAGATAAAGTAGTTGTAATTACAGGTGGATCAACAGGTTTAGGACGCGCAA
TGGCTGTTCGTTTCGGTCAAGAAGAAGCAAAAGTTGTTATTAACTATTACAACAATGAAGAAGAAGCT
TTAGATGCGAAAAAAGAAGTAGAAGAAGCAGGCGGACAAGCAATCATCGTTCAAGGCGACGTAACA
AAAGAAGAAGATGTTGTAAACCTTGTTCAAACAGCTATTAAAGAATTCGGTACATTAGACGTTATGAT
TAATAACGCTGGTGTTGAAAACCCAGTTCCTTCTCATGAGTTATCTTTAGACAACTGGAATAAAGTTAT
TGATACAAACTTAACAGGTGCATTCTTAGGAAGCCGTGAAGCAATCAAATATTTTGTTGAAAACGACA
TTAAAGGAAACGTTATTAACATGTCTAGTGTTCATGAAATGATTCCTTGGCCATTATTTGTTCATTACG
CAGCAAGTAAAGGCGGTATGAAACTAATGACGGAAACATTGGCTCTTGAATATGCGCCAAAAGGTAT
CCGCGTAAATAACATTGGACCAGGTGCGATGAACACACCAATTAACGCAGAGAAATTTGCAGATCCT
GTACAACGTGCAGACGTAGAAAGCATGATTCCAATGGGTTACATCGGTAAACCAGAAGAAGTAGCA
GCAGTTGCAGCATTCTTAGCATCATCACAAGCAAGCTATGTAACAGGTATTACATTATTTGCTGATGG
TGGTATGACGAAATACCCTTCTTTCCAAGCAGGAAGAGGCTAA
SEQ ID Nr. 20:

MYTDLKDKVVVITGGSTGLGRAMAVRFGQEEAKVVINYYNNEEEALDAKKEVEEAGGQAIIVQGDVTKEED
VVNLVQTAIKEFGTLDVMINNAGVENPVPSHELSLDNWNKVIDTNLTGAFLGSREAIKYFVENDIKGNVIN

MSSVHEMIPWPLFVHYAASKGGMKLMTETLALEYAPKGIRVNNIGPGAMNTPINAEKFADPVQRADVES
MIPMGYIGKPEEVAAVAAFLASSQASYVTGITLFADGGMTKYPSFQAGRG

Die hier angeführte Glucose-Dehydrogenase zur Kofaktor-Regeneration umfasst vorzugsweise eine Aminosäuresequenz, die eine Identität zu SEQ ID Nr. 20 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Glucose-Dehydrogenase zur Kofaktor-Regeneration die Aminosäuresequenz SEQ ID Nr. 20 oder besteht aus dieser.

Alternativ umfasst die Glucose-Dehydrogenase zur Kofaktor-Regeneration vorzugsweise eine Aminosäuresequenz, die durch eine Nukleinsäure kodiert wird, die eine Identität zu SEQ ID Nr. 19 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Glucose-Dehydrogenase zur Kofaktor-Regeneration kodiert, die Nukleinsäuresequenz SEQ ID Nr. 19 oder besteht aus dieser.

Ein weiterer Aspekt der vorliegenden Erfindung betrifft die Verwendung einer Glucose-Dehydrogenase zur Kofaktor-Regeneration, wobei die Glucose-Dehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 20 von mindestens 80% aufweist,
ii) einer Aminosäuresequenz, die durch eine Nukleinsäure kodiert wird, die eine Identität zu SEQ ID Nr. 19 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 19 bindet.

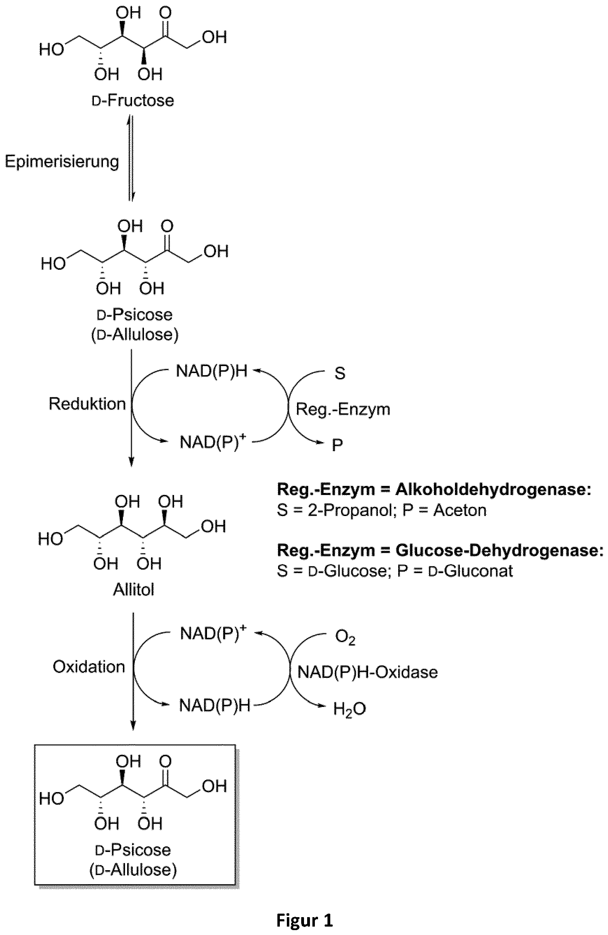

Die zur Kofaktor-Regeneration eingesetzte NAD(P)H-Oxidase (siehe Figur 1) kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Eine besonders bevorzugt eingesetzte H₂O-bildende NAD(P)H-Oxidase umfasst oder besteht vorzugsweise aus eine Aminosäuresequenz, die ausgewählt ist aus der Gruppe bestehend aus:
i) einer Aminosäuresequenz, die eine Identität zu SEQ ID Nr. 16 oder SEQ ID Nr. 14 von mindestens 80% aufweist,
ii) einer Aminosäuresequenz, die durch eine Nukleinsäure kodiert wird, die eine Identität zu SEQ ID Nr. 15 oder SEQ ID Nr. 13 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 15 oder SEQ ID Nr. 13 bindet.

SEQ ID Nr. 13: 
ATGAGCAAAATTGTTATCGTGGGTGCAAATCATGCAGGCACCGCAGCAATTAATACCATTCTGGATAATTA
TGGCAGCGAAAATGAAGTGGTTGTGTTTGATCAGAATAGCAACATTAGCTTTCTGGGTTGTGGTATGGCAC
TGTGGATTGGTAAACAAATTAGCGGTCCGCAGGGTCTGTTTTATGCAGATAAAGAAAGCCTGGAAGCAAA
AGGTGCCAAAATCTATATGGAAAGTCCGGTTACCGCCATTGATTATGATGCAAAACGTGTTACCGCACTGG
TTAATGGTCAAGAACATGTTGAAAGCTACGAGAAACTGATTCTGGCAACCGGTAGCACCCCGATTCTGCCT
CCGATTAAAGGTGCAGCCATTAAAGAAGGTAGTCGCGATTTTGAAGCAACCCTGAAAAATCTGCAGTTCGT
GAAACTGTATCAGAATGCCGAAGATGTGATTAACAAACTGCAGGATAAAAGCCAGAATCTGAATCGTATT
GCAGTTGTTGGTGCAGGTTATATTGGTGTTGAACTGGCAGAAGCATTTAAACGTCTGGGTAAAGAAGTGA
TTCTGATTGACGTTGTTGATACCTGTCTGGCAGGTTATTATGATCAGGATCTGAGCGAAATGATGCGTCAG
AATCTGGAAGATCATGGTATCGAACTGGCATTTGGTGAAACCGTTAAAGCAATTGAAGGTGATGGTAAAG
TGGAACGTATTGTTACCGATAAAGCAAGCCATGATGTGGATATGGTTATTCTGGCAGTTGGTTTTCGTCCG
AATACAGCACTGGGTAATGCAAAACTGAAAACCTTTCGTAATGGTGCCTTTCTGGTGGATAAAAAACAAGA
AACCAGCATCCCGGATGTTTATGCAATTGGTGATTGTGCAACCGTGTATGATAATGCCATTAACGACACCA
ACTATATTGCACTGGCAAGCAATGCACTGCGTAGCGGTATTGTTGCAGGTCATAATGCAGCCGGTCATAAA
CTGGAAAGTCTGGGTGTTCAGGGTAGCAATGGTATTTCAATTTTTGGCCTGAATATGGTTAGCACCGGTCT
GACCCAAGAAAAAGCCAAACGTTTTGGTTATAATCCGGAAGTTACCGCCTTTACCGATTTTCAGAAAGCCA
GCTTTATCGAGCATGATAACTATCCGGTTACGCTGAAAATTGTGTATGACAAAGATAGCCGTCTGGTTCTG
GGTGCACAGATGGCCAGCAAAGAAGATATGAGCATGGGTATTCACATGTTTAGCCTGGCCATTCAAGAGA
AAGTTACCATTGAACGTCTGGCCCTGCTGGATTATTTCTTTCTGCCGCATTTTAATCAGCCGTACAACTATAT
GACCAAAGCAGCACTGAAAGCCAAATAA
SEQ ID Nr. 14: 
MSKIVIVGANHAGTAAINTILDNYGSENEVVVFDQNSNISFLGCGMALWIGKQISGPQGLFYADKESLEAKGAKI
YMESPVTAIDYDAKRVTALVNGQEHVESYEKLILATGSTPILPPIKGAAIKEGSRDFEATLKNLQFVKLYQNAEDVI
NKLQDKSQNLNRIAVVGAGYIGVELAEAFKRLGKEVILIDVVDTCLAGYYDQDLSEMMRQNLEDHGIELAFGET
VKAIEGDGKVERIVTDKASHDVDMVILAVGFRPNTALGNAKLKTFRNGAFLVDKKQETSIPDVYAIGDCATVYD
 
NAINDTNYIALASNALRSGIVAGHNAAGHKLESLGVQGSNGISIFGLNMVSTGLTQEKAKRFGYNPEVTAFTDF
QKASFIEHDNYPVTLKIVYDKDSRLVLGAQMASKEDMSMGIHMFSLAIQEKVTIERLALLDYFFLPHFNQPYNY
MTKAALKAK
SEQ ID Nr. 15: 
ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
AATCAAAAAATGCTAAAAAGATTGTCATTGTTGGTGGTGGCTATATTGGAATTGAATTAGTTGAGGCATTT
GCAGAATCTGGCAAGCAAGTGACGCTAGTTGATGGATTAGATCGTATTTTAAACAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT
CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA
SEQ ID Nr. 16:

MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIGIELVEAFAESGKQVTLVDGLDRILNKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK

Die bevorzugt verwendete H₂O-bildende NAD(P)H-Oxidase umfasst oder besteht vorzugsweise aus einer Aminosäuresequenz, die eine Identität zu SEQ ID Nr. 16 oder SEQ ID Nr. 14 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die H₂O-bildende NAD(P)H-Oxidase die Aminosäuresequenz SEQ ID Nr. 16 oder SEQ ID Nr. 14 oder besteht aus dieser.

Alternativ umfasst die H₂O-bildende NAD(P)H-Oxidase vorzugsweise eine Aminosäuresequenz, die durch eine Nukleinsäure kodiert wird, die eine Identität zu SEQ ID Nr. 15 oder SEQ ID Nr. 13 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die H₂O-bildende NAD(P)H-Oxidase kodiert, die Nukleinsäuresequenz SEQ ID Nr. 15 oder SEQ ID Nr. 13 oder besteht aus dieser.

Ein weiterer Aspekt der vorliegenden Erfindung betrifft die die Verwendung einer H₂O-bildenden NAD(P)H-Oxidase zur Kofaktor-Regeneration (NAD(P)H zu NAD(P)⁺), welche eine Aminosäuresequenz umfasst oder daraus besteht, die ausgewählt ist aus der Gruppe bestehend aus:
i) einer Aminosäuresequenz, die eine Identität zu SEQ ID Nr. 16 oder SEQ ID Nr. 14 von mindestens 80% aufweist,
ii) einer Aminosäuresequenz, die durch eine Nukleinsäure kodiert wird, die eine Identität zu SEQ ID Nr. 15 oder SEQ ID Nr. 13 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 15 oder SEQ ID Nr. 13 bindet.

Die hier vorgestellte enzymatische Strategie in Kombination mit der Kofaktor-Regeneration ermöglicht einen biokatalytischen, umweltfreundlichen und hocheffizienten Produktionsprozess zur Herstellung von D-Psicose.

Oxidoreduktasen können zur Bildung von L-Psicose aus Allitol eingesetzt werden. Eine Oxidoreduktase zur Bildung von L-Psicose aus Allitol ist vorzugsweise eine Mannitol-Dehydrogenase-artige Short-Chain-Dehydrogenase/Reduktase. Es hat sich überraschenderweise gezeigt, dass Mannitol-Dehydrogenase-artige Short-Chain-Dehydrogenasen/Reduktasen in der Lage sind, Allitol in Anwesenheit des Kofaktors NAD(P)⁺ zu L-Psicose umzusetzen. Dementsprechend betrifft ein weiterer Aspekt der vorliegenden Erfindung ein Verfahren zur Herstellung von L-Psicose umfassend den Schritt des Behandelns von Allitol mit einer Oxidoreduktase, vorzugsweise einer Mannitol-Dehydrogenase-artigen Short-Chain-Dehydrogenase/Reduktase, in Anwesenheit des Kofaktors NAD(P)⁺.

Eine Oxidoreduktase zur Bildung von L-Psicose aus Allitol umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

SEQ ID Nr. 1: 
ATGTCTCAAGCTATCTCAGCAATCAACGAAAGAGTTGGCCCATTGCCCACTAAAGCACCCCAACTTTCAAA
AAACGTATTAGACCTCTTTTCCTTGAAAGGCAAGGTAGCATCGGTGACAGGTTCATCGAAGGGAATAGG
CTTTGCTGTCGCAGAAGCATACGCTCAAGCAGGGGCTGATGTTGCTTTGTGGTACAATTCAAGTCCTGTC
GAGGATAAGGTAAAATACTTGAAAGAGACCTACGGCGTGAAAGTCAAAGCCTACAAGTGTAACGTGGG
AAACAGCGAGGAGGTTGAGAAAATAGTAGACCAAATTGCAGAGGACTTCGGCACAATTGACGTTTTTGT
TGCTAATGCGGGCATTGCTTGGAGCGAAGGTAGATCTCTCGAGGTTAAGGGTTACGACGCTTGGCAAAA
AATTGTCGACTACGACTTGAGCAGTGTATACTACTGTGCTAAGGCAGTGGGTAAGATCTTCCAAGAAAAG
GGCTCCGGTTCGTTAATCCTCACCGCATCCATGTCCGGACACATTGTGAATGTTCCTCAAATGCAGGCACC
ATACAATGCGGCCAAGGCGGCTGTGATCCATCTAGGAAAATCTTTGGCTGTCGAATGGGCTCCATTTGCC
AGGGTCAACACCGTTTCTCCTGGCTATATTGCCACTGATATTGGTGAATTTGTGCTGCCCGATGAGAAGA
AAAAGTGGTGGTCATTGACACCCTTGGGTAGAGAAGGTCTTCCTCAAGAACTCACTGGTGCATACTTGTA
TTTTGCTTCTAACGCATCGACCTACACTACTGGGGCTGACTTGGTCGTAGACGGTGGCTACACTGTACCAT
AA
SEQ ID Nr. 2:

MSQAISAINERVGPLPTKAPQLSKNVLDLFSLKGKVASVTGSSKGIGFAVAEAYAQAGADVALWYNSSPVEDK
VKYLKETYGVKVKAYKCNVGNSEEVEKIVDQIAEDFGTIDVFVANAGIAWSEGRSLEVKGYDAWQKIVDYDLS
SVYYCAKAVGKIFQEKGSGSLILTASMSGHIVNVPQMQAPYNAAKAAVIHLGKSLAVEWAPFARVNTVSPGYI
ATDIGEFVLPDEKKKWWSLTPLGREGLPQELTGAYLYFASNASTYTTGADLVVDGGYTVP

Die NAD(P)H-abhängige Oxidoreduktase zur Reduktion der ersten D-Psicose zu Allitol umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Besonders geeignet zur Reduktion der ersten D-Psicose zu Allitol bzw. D-Psicose zu Allitol im Allgemeinen ist eine Oxidoreduktase, deren Aminosäuresequenz mindestens 80% ident zu SEQ ID Nr. 4 ist bzw. welche durch eine Nukleinsäure kodiert wird, die eine Identität zu SEQ ID Nr. 3 von mindestens 80% aufweist bzw. unter stringenten Bedingungen an ein Nukleinsäuremolekül mit der Nukleinsäuresequenz SEQ ID Nr. 3 bindet. Diese Oxidoreduktase kann zudem überraschenderweise zur Oxidation von Allitol zu D-Psicose eingesetzt werden.

Die NAD(P)⁺-abhängige Oxidoreduktase zur Bildung von D-Psicose aus Allitol umfasst oder besteht aus einer Aminosäuresequenz, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet.

SEQ ID Nr. 3: 
ATGACACAGTCTCTTCAGGGCAAGATCGTCGCCATTACTGGCGCGGCTTCGGGCATTGGCCTCGAATGC
GCCCGCTATCTCATCGAAGCTGGCGCGGTGGTCTATCTTCTGGACCGTGACGCCAAAACTCTCGAAGAC
AAAACGGCAGAACTCGGCAGCCAGGCCCATGCGATCATCGTCGATCTCTTCGACTACAAAACCGTAGAT
GCTGCGGTCGCGCAGATCGTTGAGGAGCAGGGCCGGATCGATGTTTTCCACGCCAACGCCGGCGCGTA
TGTGGGCGGCAATGTCTGGGAAGGGGATCCCGATAGCTGGGATGCGATGCTGCACCTGAACATCAAT
GCAGCTTTCCGCTCGGTCCGTGCCGTGCTGCCGCAGATGATGAAGCAGGAAAGTGGCGATATCGTCAT
GACCAGCTCGATCGCGGGCATGATCCCCATCATGGCCGAGCCGATCTACACGGCCTCGAAACATGCCG
TGCAGGCATTCGTACATACGGTGCGCCGACAGGTCGCGAAGTATGGAATTCGTGTGGGTGCAATCCAG
CCTGGTCCTGTAGTCACGCCTTTGCTGAAAGACTGGGATCAGGCCCGTCTTGAAGCCAACATCAAAGCG
 
GGTGCCCTGATGGAAGCCAAGGAAGTCGCCGAAGCCCTGATCTTCATCCTGACACGTTCAAAGGGTGT
CATGGTGCGGGATCTGGTCGTTCTGCCACATAACTTCGACGCCTAAGCTTAAGCGGCCGCACTCGAGCA
CCACCACCACCACCACTGAGATCCGGCTGCTAA
SEQ ID Nr. 4: 
MTQSLQGKIVAITGAASGIGLECARYLIEAGAVVYLLDRDAKTLEDKTAELGSQAHAIIVDLFDYKTVDAAVA
QIVEEQGRIDVFHANAGAYVGGNVWEGDPDSWDAMLHLNINAAFRSVRAVLPQMMKQESGDIVMTSSI
AGMIPIMAEPIYTASKHAVQAFVHTVRRQVAKYGIRVGAIQPGPVVTPLLKDWDQARLEANIKAGALMEA
KEVAEALIFILTRSKGVMVRDLVVLPHNFDA
SEQ ID Nr. 5: 
ATGAGCACACCGGAAAATCTGAGCTTTGTGCTGCAGAAACCGTTTGATGTGAAATTTGAAGATCGTCCG
ATTCCGAAACTGAGCGATCCGTATAGCGTTAAAATTCAGGTGAAAAAAACCGGCATTTGCGGTAGTGA
TGTTCACTATTTCACCCATGGTGCAATTGGTGATTTTGTTGTTAAAGCACCGATGGTTCTGGGTCATGAA
AGCAGCGGTGTTGTTCTGGAAGTTGGTAGCGAAGTTAAAAGCCTGAAAGTTGGTGATCGTGTTGCAAT
GGAACCGGGTGTTCCGAGCCGTCATAGTGATGAGTATAAAAGCGGTCGTTATAATCTGTGTCCGCACA
TGGCATTTGCAGCAACCCCTCCGTATGATGGCACCCTGTGTAAATACTATATTCTGCCGGAAGATTTCTG
CGTTAAACTGCCGGAACATGTTAGCCTGGAAGAAGGTGCACTGGTTGAACCGCTGAGCGTTGCAGTTC
ATAGCAGCAAACTGGGTAACATTAAACCGGGTAGCCATGTTGCAATTTATGGTGCAGGTCCGGTTGGT
CTGCTGGTTGCAGCAGTTGCAAGCGCATTTGGTGCAGAAAGCGTTACCATTATTGATCTGGTTGAAAGC
CGTCTGAATCTGGCAAAAGAACTGGGTGCAACCGCAACCGTTCAGGTTGATTTTAAAGATACCCCGAA
AGAAAGCGCAGCAAAAGTTGTTGCAGCAAATAATGGCATTGCACCGGATGTTGTTATTGATGCAAGCG
GTGCAGAAGCAAGCATTAATTCAGCCATTAATGCAATTCGTCCGGGTGGCACCTATGTTCAGGTGGGTA
TGGGTAAACCGGATGTGAGCTTTCCGATTGCAACCCTGATTGGTAAAGAACTGACCGTTAAAGGTAGC
TTTCGTTATGGTTATGGTGATTATCCGCTGGCAGTTAGCCTGCTGGCAAGCGGTAAAGTTAATGTGAAA
AAACTGATCACCCATGAAGTGAAATTCGAGGATGCAGCAGAAGCATTTCAGCTGGTTCGTGATGGTAA
AGCCATTAAATGTATTATCAACGGTCCGGAATAA
SEQ ID Nr. 6: 
MSTPENLSFVLQKPFDVKFEDRPIPKLSDPYSVKIQVKKTGICGSDVHYFTHGAIGDFVVKAPMVLGHESSGV
VLEVGSEVKSLKVGDRVAMEPGVPSRHSDEYKSGRYNLCPHMAFAATPPYDGTLCKYYILPEDFCVKLPEHV
SLEEGALVEPLSVAVHSSKLGNIKPGSHVAIYGAGPVGLLVAAVASAFGAESVTIIDLVESRLNLAKELGATAT
VQVDFKDTPKESAAKVVAANNGIAPDVVIDASGAEASINSAINAIRPGGTYVQVGMGKPDVSFPIATLIGKE
LTVKGSFRYGYGDYPLAVSLLASGKVNVKKLITHEVKFEDAAEAFQLVRDGKAIKCIINGPE
SEQ ID Nr. 7: 
ATGAATAACAACCTGCCGAAAACCATGAAAGCAGCAGTTATGCATGGCACCCGTGAAATTAGCATTGA
AACCCTGCCGATTCCGCAGATTGATGAAAATGAAGTTCTGATCAAAGTTATGGCCGTTGGTATTTGTGG
TAGCGATCTGCACTATTACACCCAGGGTCGTATTGGTAAATACAAAGTGGAAAAACCGTTTATCCTGGG
TCATGAATGTAGCGGTGAAGTTGTTGCAATTGGTAGCGCAGTTGAACGTTTTCGTGTTGGTGATCGTGT
TGCCGTTGAACCGGGTGTTACCTGTGGTCATTGTGAAGCATGTAAAGAGGGTCGTTATAATCTGTGTCC
GGATGTTCAGTTTCTGGCAACCCCTCCGGTTGATGGTGCATTTGTTCAGTATATCAAAATGCGCCAGGA
TTTCGTTTTTCTGATTCCGAATAGCCTGAGCTATGAAGATGCAGCACTGATTGAACCGTTTAGCGTGGG
TATTCATGCAGCAACCCGTACCAAACTGCAGCCTGGTAGCACCATTGCAATTATGGGTATGGGTCCGGT
TGGTCTGATGGCAGTTGCAGCAGCAAAAGCATTTGGTGCAAGCACCATTATTGCAACCGATCTGGAAC
CGCTGCGTCTGGAAGCAGCCAAACGTATGGGTGCAACCCATGTTATTAACATTCGTGAACAGGATCCG
CTGAACGAGATTAAAAACATTACCGAAAATGTGGGTGTTGATGTTGCATGGGAAACCGCAGGTAATCC
GAAAGCACTGCAGAGCAGCCTGAGCAGCATTCGTCGTGGTGGTAAACTGGCAATTGTTGGTCTGCCGA
GCCAGAGCGATATTCCGCTGGATGTTCCGTTTATTGCCGATAATGAAATCGATATCTATGGCATCTTTCG
CTATGCAAACACCTATCCGAAAGGCATCAAATTTCTGACCAGCGGTGCAATTGATACCAAAAATCTGGT
TACCGATCGTTATCCGCTGGCAGGTACACGTGAAGCAATGGAACGTGCACTGAATTTCAAAAACGAAT
GCCTGAAAATCATCGTGTATCCGAACGAATAA
SEQ ID Nr. 8: 
MNNNLPKTMKAAVMHGTREISIETLPIPQIDENEVLIKVMAVGICGSDLHYYTQGRIGKYKVEKPFILGHECS
GEVVAIGSAVERFRVGDRVAVEPGVTCGHCEACKEGRYNLCPDVQFLATPPVDGAFVQYIKMRQDFVFLIP
NSLSYEDAALIEPFSVGIHAATRTKLQPGSTIAIMGMGPVGLMAVAAAKAFGASTIIATDLEPLRLEAAKRMG
ATHVINIREQDPLNEIKNITENVGVDVAWETAGNPKALQSSLSSIRRGGKLAIVGLPSQSDIPLDVPFIADNEI
DIYGIFRYANTYPKGIKFLTSGAIDTKNLVTDRYPLAGTREAMERALNFKNECLKIIVYPNE
SEQ ID Nr. 9: 
ATGACCTCTCCTCTCCAGGGTAAGATAGCCGCCATCACGGGCGGGGCTTCGGGCATCGGCCTCGAATG
TGTCCGCCAGATCGCCGCAAGTGGTGCCACGGTTTATATTCTCGACCGCGACCATCAGGCGCTCGACAA
GGCGCGCGAAGAATTGGGCGAGCGCGTTCATACCATCGAGGTCGATCTCTTCCGTTACGAAACGGTCG
ATCGCGCCATCGAAACCATCGTGTCCGAACAAGGACGCATCGACATTCTCCATGTCAATGCGGGCGCGT
ATATCGGCGGCAATGTCTGGGAAGGCGATCCCGATAAATGGGACAAGATGCTGAATCTCAACATCAAC
GCCGCCTTCCGTTCCGCCCGCGCCGTCATGCCCGCCATGATGAAGCAGAAAAGCGGCGATATCATCATG
ACAAGCTCGATCGCAGGCATCGTCCCGATCCCGGCGGAGCCGATCTACACGGCTTCCAAACATGCGGT
GCAAGCCTTCGCTCACACCATACGCCGCCAGTTGGCCCCGTTCGGCATCCGCGTCGGCGCCATCCAGCC
 
CGGCCCGGTCGTCACGCCCTTGCTCAATGATTGGGACCCCGAGCGCCTTAAAGCCAATATCGAGGCTG
GCGCCATGATGCAGCCTTCTGACGTCGCCGAAGCCGTGGTTTTCATGCTGTCCCGCCGCAAGGGAACG
GTAATCCGCGACTTGGTTCTGTTACCCCATTCTTTCGACGTCTAA
SEQ ID Nr. 10: 
MTSPLQGKIAAITGGASGIGLECVRQIAASGATVYILDRDHQALDKAREELGERVHTIEVDLFRYETVDRAIET
IVSEQGRIDILHVNAGAYIGGNVWEGDPDKWDKMLNLNINAAFRSARAVMPAMMKQKSGDIIMTSSIAGI
VPIPAEPIYTASKHAVQAFAHTIRRQLAPFGIRVGAIQPGPVVTPLLNDWDPERLKANIEAGAMMQPSDVA
EAVVFMLSRRKGTVIRDLVLLPHSFDV
SEQ ID Nr. 11: 
ATGGCTATATCTCTCGAAAACAACGTAGCTGCAATTACAGGTGCCGCTTCAGGTATCGGTCTCGAATGT
GCACGCACACTGATCAAAGCAGGCGCTAAAGTTGTCCTCATTGACCGAGCAGAAGATAGACTAAATCA
ATTGGTCGCAGAATTAGGTGAAAATGCAATTCCATTAGTTATCGATTTAATGAAACCAGAACAAGTCGA
TGGCATGTTAGCGCGTATTATCGAAAAGGCAGGCAGATTAGATATCTTTCATGCTAATGCTGGAGCTTA
CATTGGTGGGCCCGTAGCCGAAGGCGATCCCGATGTTTGGGATAAAGTCCTAAATTTAAATGTTAATGC
CGCATTCCGCTGTGTTCGCGCAGTTCTACCACACTTTATCGCACAAAAGTCAGGCGATATTCTATTCACC
AGCTCTATCGCTGGTATGGTTCCCGTAATTTGGGAGCCTATTTACACGGCATCAAAATTTGCGGTTCAA
GCATTCGTTCATTCTACTCGCCGTCAGGTTTCTGAACACGGTGTCCGTGTTGGTGCTGTATTACCTGGTC
CTGTTGTTACTGCGTTATTAGATGATTGGCCAAAAGAAAAACTTGAAGAAGCTTTAGCTAACGGTAGTT
TAATGCAACCCATTGAAGTTGCTGAGGCTGTTCTATTCATGCTGACGCGTCCAAGAAATATTACAATTCG
CGATTTAGTTATTTTACCCAATAGTGTTGACCTCTAA
SEQ ID Nr. 12:

MAISLENNVAAITGAASGIGLECARTLIKAGAKVVLIDRAEDRLNQLVAELGENAIPLVIDLMKPEQVDGMLA
RIIEKAGRLDIFHANAGAYIGGPVAEGDPDVWDKVLNLNVNAAFRCVRAVLPHFIAQKSGDILFTSSIAGMV
PVIWEPIYTASKFAVQAFVHSTRRQVSEHGVRVGAVLPGPVVTALLDDWPKEKLEEALANGSLMQPIEVAE
AVLFMLTRPRNITIRDLVILPNSVDL

Die hier angeführten Oxidoreduktasen zur Reduktion von D-Psicose zu Allitol und/oder zur Oxidation von Allitol zu D-Psicose und/oder zur Bildung von L-Psicose aus Allitol umfassen vorzugsweise eine Aminosäuresequenz, die eine Identität zu SEQ ID Nr. 2, SEQ ID Nr. 4, SEQ ID Nr. 6, SEQ ID Nr. 8, SEQ ID Nr. 10 oder SEQ ID Nr. 12 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Reduktion von D-Psicose zu Allitol und/oder zur Oxidation von Allitol zu D-Psicose die Aminosäuresequenz SEQ ID Nr. 4 oder besteht aus dieser, die Oxidoreduktase zur Oxidation von Allitol zu D-Psicose umfasst eine der Aminosäuresequenzen SEQ ID Nr. 6, SEQ ID Nr. 8, SEQ ID Nr. 10 oder SEQ ID Nr. 12 oder besteht aus dieser und die Oxidoreduktase zur Bildung von L-Psicose aus Allitol umfasst die Aminosäuresequenz SEQ ID Nr. 2 oder besteht aus dieser.

Alternativ umfassen die Oxidoreduktasen zur Reduktion von D-Psicose zu Allitol und/oder zur Oxidation von Allitol zu D-Psicose und/oder zur Bildung von L-Psicose aus Allitol vorzugsweise eine Aminosäuresequenz, die durch eine Nukleinsäure kodiert wird, die eine Identität zu SEQ ID Nr. 1, SEQ ID Nr. 3, SEQ ID Nr. 5, SEQ ID Nr. 7, SEQ ID Nr. 9 oder SEQ ID Nr. 11 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Reduktion von D-Psicose zu Allitol und/oder zur Oxidation von Allitol zu D-Psicose kodiert, umfasst die Nukleinsäuresequenz SEQ ID Nr. 3 oder besteht aus dieser, die Oxidoreduktase zur Oxidation von Allitol zu D-Psicose kodiert, umfasst die Nukleinsäuresequenz SEQ ID Nr. 5, SEQ ID Nr. 7, SEQ ID Nr. 9 oder SEQ ID Nr. 11 oder besteht aus dieser und die Oxidoreduktase zur Bildung von L-Psicose aus Allitol kodiert, umfasst die Nukleinsäuresequenz SEQ ID Nr. 1 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N =-4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = - 4, und einen Vergleich beider Stränge.

Alternativ umfassen die Oxidoreduktasen zur Reduktion von D-Psicose zu Allitol und/oder zur Oxidation von Allitol zu D-Psicose und/oder zur Bildung von L-Psicose aus Allitol vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein Aspekt der vorliegenden Erfindung betrifft die Verwendung einer Oxidoreduktase zur Reduktion von D-Psicose zu Allitol und/oder zur Oxidation von Allitol zu D-Psicose, wobei die Oxidoreduktase eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Ein weiterer Aspekt der vorliegenden Erfindung betrifft die Verwendung einer Oxidoreduktase zur Oxidation von Allitol zu D-Psicose, dadurch gekennzeichnet, dass die Oxidoreduktase eine Aminosäuresequenz umfasst, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet.

Die erfindungsgemäßen Oxidoreduktasen benötigen je nach Reaktion (Reduktion oder Oxidation) entsprechende Kofaktoren, wie oben angeführt.

### Materialien

D-Psicose wurde von TCI und Hunan Garden Naturals Inc. (China), Allitol wurde von TCI, D-Fructose, NADPH-Tetranatriumsalz, und Methanol wurden von PanReac AppliChem (ITW Reagents), D-Glucose, Natriumgluconat, IPTG (Isopropyl-β-D-thiogalactopyranosid) wurden von Sigma-Aldrich, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen und Spenderorganismen für die in den Beispielen verwendeten Enzyme (SDR = Oxidoreduktase aus Short-Chain-Dehydrogenase/Reduktase-Familie).**

| **Enzymtyp (EC-Klasse)** | **katalysierte Reaktion** | **Spenderorganism US** | **Literatur/SEQ ID Nr.** |
|---|---|---|---|
| D-Psicose-3-Epimerase (EC 5.1.3.30) | D-Fructose → D-Psicose | *Clostridium cellulolyticum* H10 | (Mu et al., 2011; Chan et al., 2012) |
| Ribitol-Dehydrogenase (EC 1.1.1.56) | D-Psicose ↔ Allitol | *Klebsiella pneumoniae* | (Takeshita et al., 2000; Sequenz in NCBI Protein Database: WP_265716617.1) |
| SDRI | D-Psicose ↔ Allitol | *Gluconobacter frateurii* (DSM 7146) | (NCBI Protein database: WP_063903495.1); SEQ ID Nr. 4 |
| Xylitol-Dehydrogenase (XDH; EC 1.1.1.9) | Allitol → D-Psicose | *Galactocandida mastotermitis* (*Candida* sp. HA167) | (Habenicht et al., 1999); SEQ ID Nr. 6 |
| NAD(P)-abhängige Alkohol-Dehydrogenase | Allitol → D-Psicose | *Priestia megaterium* | (NCBI Protein database: WP_013084280.1); SEQ ID Nr. 8 |
| SDR II | Allitol → D-Psicose | *Kozakia baliensis* | (NCBI Protein Database: WP_070401870.1); SEQ ID Nr. 10 |
| SDR III | Allitol → D-Psicose | *Providencia heimbachae* | (NCBI Protein Database: WP_068907433.1); SEQ ID Nr. 12 |
| Mannitol-Dehydrogenase-artige Short-Chain-Dehydrogenase/Redukta se | Allitol → L-Psicose | *Millerozyma farinosa (Pichia sorbitophila)* CBS 7064 | (Louis et al., 2012); SEQ ID Nr. 2 |
| Alkoholdehydrogenase (ADH; EC 1.1.1.1) | 2-Propanol → Aceton | *(Geo-)Bacillus stearothermophil* us NCA1503 | (Sakoda & Imanaka, 1992); SEQ ID Nr. 18 |
| NADH-Oxidase I (EC 1.6.3.4) | NADH → NAD⁺ | *Streptococcus mutans* | (Matsumoto et al., 1996); SEQ ID Nr. 14 |
| NADH-Oxidase II (EC 1.6.3.4) | NADH → NAD⁺ | *Carnobacterium divergens* | SEQ ID Nr. 16 |
| Glucose-Dehydrogenase (GDH; EC 1.1.1.47) | D-Glucose → D-Gluconat (via D-Gluconolacton) | *Priestia megaterium* | (NCBI Protein Database: MDQ0804260.1); SEQ ID Nr. 20 |

### Analytische Methoden

### High Performance Liquid Chromatography

Zur Quantifizierung von D-Psicose, D-Fructose, D-Glucose sowie Allitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### High Performance Anion Exchange Chromatography

Zur Quantifizierung von D-Gluconsäure/D-Gluconat mittels HPAEC (High Performance Anion Exchange Chromatography) wurde ein Dionex ICS6000 System mit AS-AP Autosampler verwendet. Die Messung erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC Anion Trap Column vorbehandelt.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Herstellung von D-Psicose aus D-Fructose - Kofaktor-Regeneration mit ADH und 2-Propanol

Die Reaktion wurde in einem Labfors 5 Tisch-Bioreaktor (Infors AG) durchgeführt. Als Gefäß wurde ein Glasreaktor (Volumen 3,4 l) mit aufgesetztem Rührwerk und pH-Elektrode verwendet. Die pH-Kontrolle erfolgte durch die Zudosierung von 1M NaOH oder 1M H₂SO₄.

Zu Beginn wurden 50 ml einer D-Fructose-Lösung (500 g/l), 246,1 ml deionisiertes Wasser sowie 96 ml eines 200 mM TEA-HCl-Puffers (pH 8) im Reaktor vorgelegt und unter Rühren auf 35 °C gebracht.

Zum Start der Reaktion wurden 25 ml D-Psicose-3-Epimerase-Lysat zugesetzt. Danach wurden 25 ml SDR I-Lysat, 4 kU Alkoholdehydrogenase-Lysat, 10 ml einer 10 mM NAD⁺-Lösung sowie 40 ml 2-Propanol eingebracht.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (Rl-Detektion) vermessen.

Nach 23 h Laufzeit wurden 15 ml, nach 50 h wurden 20 ml und nach 77 h wurden 15 ml 2-Propanol nachdosiert.

Nach 74 h wurden 5 ml D-Psicose-3-Epimerase-Lysat, nach 77 h wurden 15 ml SDR I-Lysat und 2,4 kU Alkoholdehydrogenase-Lysat zugegeben.

Nach 97 h wurden 94% der D-Fructose (50 g/l) zu Allitol umgesetzt (gefundene Konzentration: 42 g/l).

Danach wurde der gesamte Reaktorinhalt für 60 min auf 70 °C erhitzt (Deaktivierung der D-Psicose-3-Epimerase) und nach Abkühlen auf 24 °C wurden 25 ml Xylitol-Dehydrogenase-Lysat, 10 kU NADH-Oxidase-Lysat sowie 10 ml einer 10 mM NAD⁺-Lösung zugesetzt.

Allitol wurde innerhalb von 3 h vollständig zu D-Psicose oxidiert. Der Reaktorinhalt wurde auf 70 °C erhitzt, der pH-Wert auf 4 eingestellt und für 30 min bei 70 °C gerührt. Die Enzyme wurden über eine Glasfritte (P3) abfiltriert.

Im Filtrat wurden 34,7 g/l D-Psicose detektiert.

Das Filtrat wurde bis zu einer D-Psicose-Konzentration von 520 g/l am Rotationsverdampfer aufkonzentriert.

Das Beispiel 1 zeigt, dass die Epimerase durch Hitzeeinwirkung (im Eintopf-Verfahren) denaturiert werden kann und der entstandene Niederschlag die weitere Reaktionsführung nicht stört.

### Beispiel 2

### Herstellung von D-Psicose aus D-Fructose - Kofaktor-Regeneration mit GDH und D-Glucose

Die Reaktion wurde in einem Multifors Tisch-Bioreaktor (Infors AG) durchgeführt. Als Gefäß wurde ein Glasreaktor (Volumen 1 l) mit aufgesetztem Rührwerk und pH-Elektrode verwendet. Die pH-Kontrolle erfolgte durch die Zudosierung von 5M NaOH oder 1M H₂SO₄.

Zu Beginn wurden 17,5 g D-Fructose und 17,5 g D-Glucose (finale Konzentration je 50 g/l), 217,8 ml deionisiertes Wasser sowie 26,5 ml eines 500 mM Kaliumphosphat-Puffers (pH 7,5) im Reaktor vorgelegt und unter Rühren auf 35 °C gebracht.

Zum Start der Reaktion wurden 17,5 ml D-Psicose-3-Epimerase-Lysat zugesetzt. Danach wurden 24,5 ml SDR I-Lysat, 0,4 kU Glucose-Dehydrogenase-Lysat sowie 3,5 ml einer 10 mM NAD⁺-Lösung eingebracht.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen. Für die HPAEC-Messungen (Leitfähigkeitsdetektion) wurde der klare Überstand 1:250 verdünnt.

Nach 16 h konnten im Reaktor nur mehr Allitol und D-Gluconat detektiert werden.

Danach wurde der gesamte Reaktorinhalt für 60 min auf 70 °C erhitzt (Deaktivierung der D-Psicose-3-Epimerase) und nach Abkühlen auf 30 °C wurden 17,5 ml Xylitol-Dehydrogenase-Lysat, 7 kU NADH-Oxidase-Lysat sowie 3,5 ml einer 10 mM NAD⁺-Lösung zugesetzt.

Allitol wurde innerhalb von 27 h vollständig zu D-Psicose oxidiert (gefundene Konzentration: 45 g/l). Der Reaktorinhalt wurde auf 70 °C erhitzt, der pH-Wert auf 4 eingestellt und für 30 min bei 70 °C gerührt. Die Enzyme wurden über eine Glasfritte (P3) abfiltriert.

### Literatur

Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Hough, L., & Stacey, B. E. (1996). Variation in the allitol content of Itea plants during photosynthesis. Phytochemistry, 5(1), 171-175. https://doi.org/10.1016/S0031-9422(00)85095-5
Oshima, H., Kimura, I., & Izumori, K. (2006). Psicose Contents in Various Food Products and its Origin. Food Science and Technology Research, 12(2), 137-143. https://doi.org/10.3136/fstr.12.137
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Ahmed, A., Khan, T. A., Ramdath, D. D., Kendall, C. W. C., & Sievenpiper, J. L. (2022). Rare sugars and their health effects in humans: a systematic review and narrative synthesis of the evidence from human trials, Nutrition Reviews, 80(2), 255-270. https://doi.org/10.1093/nutrit/nuab012
Chen, Z., Gao, X.-D., & Li, Z. (2022). Recent Advances Regarding the Physiological Functions and Biosynthesis of D-Allulose. Frontiers in Microbiology, 13, 881037. https://doi.org/10.3389/fmicb.2022.881037
Doner, L. W. (1979). Isomerization of D-fructose by base: Liquid-chromatographic evaluation and the isolation of D-psicose. Carbohydrate Research, 70(2), 209-216. https://doi.org/10.1016/S0008-6215(00)87101-3
Bilik, V., & Tihlärik, K. (1974). Reactions of saccharides catalyzed by molybdate ions. IX. Epimerization of ketohexoses. Chemical Papers, 28(1), 106-109. https://www.chemicalpapers.com/?id=7&paper=5533
Izumori, K., Khan, A. R., Okaya, H., & Tsumura, T. (1993). A New Enzyme, D-Ketohexose 3-Epimerase, from Pseudomonas sp. ST-24. Bioscience, Biotechnology, and Biochemistry, 57(6), 1037-1039. https://doi.org/10.1271/bbb.57.1037
Kim, N.-H., Kim, H.-J., Kang, D.-I., Jeong, K.-W., Lee, J.-K., Kim, Y., & Oh, D.-K. (2008). Conversion Shift of D-Fructose to D-Psicose for Enzyme-Catalyzed Epimerization by Addition of Borate. Applied and Environmental Microbiology, 74(10), 3008-3013. https://doi.org/10.1128/AEM.00249-08
Lim, B.-C., Kim, H.-J., & Oh, D.-K. (2009). A stable immobilized D-psicose 3-epimerase for the production of D-psicose in the presence of borate. Process Biochemistry, 44(8), 822-828. https://doi.org/10.1016/j.procbio.2009.03.017
Zhu, P., Zeng, Y., Chen, P., Men, Y., Yang, J., Yue, X., Zhang, J., Zhu, Y., & Sun, Y. (2020). A one-pot twoenzyme system on the production of high value-added D-allulose from Jerusalem artichoke tubers. Process Biochemistry, 88, 90-96. https://doi.org/10.1016/j.procbio.2019.10.006
Li, C. Li, L., Feng, Z., Guan, L., Lu, F., & Qin, H.-M. (2021). Two-step biosynthesis of D-allulose via a multienzyme cascade for the bioconversion of fruit juices. Food Chemistry, 357, 129746. https://doi.org/10.1016/j.foodchem.2021.129746
Juneja, A., Zhang, G., Jin, Y.-S., & Singh, V. (2019). Bioprocessing and technoeconomic feasibility analysis of simultaneous production of D-psicose and ethanol using engineered yeast strain KAM-2GD. Bioresource Technology, 275, 27-34. https://doi.org/10.1016/j.biortech.2018.12.025
Patel, S. N., Singh, V., Sharma, M., Sangwan, R. S., Singhal, N. K., & Singh, S. P. (2018). Development of a thermo-stable and recyclable magnetic nanobiocatalyst for bioprocessing of fruit processing residues and D-allulose synthesis. Bioresource Technology, 247, 633-639. https://doi.org/10.1016/j.biortech.2017.09.112
Yang, P., Zhu, X., Zheng, Z., Mu, D., Jiang, S., Luo, S., Wu, Y., & Du, M. (2018). Cell regeneration and cyclic catalysis of engineered Kluyveromyces marxianus of a D-psicose-3-epimerase gene from Agrobacterium tumefaciens for D-allulose production. World Journal of Microbiology and Biotechnology, 34, 65. https://doi.org/10.1007/s11274-018-2451-6
Dedania, S. R., Patel, V. R., Soni, S. S., & Patel, D. H. (2020). Immobilization of Agrobacterium tumefaciens D-psicose 3-epimerase onto titanium dioxide for bioconversion of rare sugar. Enzyme and Microbial Technology, 140, 109605. https://doi.org/10.1016/j.enzmictec.2020.109605
Van Duc Long, N., Le, T.-H., Kim, J.-I., Lee, J. W. and Koo, Y.-M. (2009). Separation of D-psicose and D-fructose using simulated moving bed chromatography. Journal of Separation Science, 32(11), 1987-1995. https://doi.org/10.1002/jssc.200800753
Li, Y., Shi, T., Han, P., & You, C. (2021). Thermodynamics-Driven Production of Value-Added D-Allulose from Inexpensive Starch by an In Vitro EnzymaticSynthetic Biosystem. ACS Catalysis, 11(9), 5088-5099. https://doi.org/10.1021/acscatal.0c05718
Wang, W., Yang, J., Sun, Y., Li, Z., & You, C. (2020). Artificial ATP-Free in Vitro Synthetic Enzymatic Biosystems Facilitate Aldolase-Mediated C-C Bond Formation for Biomanufacturing. ACS Catalysis, 10(2), 1264-1271. https://doi.org/10.1021/acscatal.9b04696
Li, Z., Li, F., Cai, L., Chen, Z., Qin, L., & Gao, X.-D. (2020). One-Pot Multienzyme Synthesis of Rare Ketoses from Glycerol. Journal of Agricultural and Food Chemistry, 68(5), 1347-1353. https://doi.org/10.1021/acs.jafc.9b06748
Xiao, Q., Niu, J., Liu, H., Liu, Y., & Zhou, X. (2019). High Conversion of D-Fructose into D-Allulose by Enzymes Coupling with an ATP Regeneration System. Molecular Biotechnology, 61, 432-441. https://doi.org/10.1007/s12033-019-00174-6
Zhang, J., Luo, W., Wang, Z., Chen, X., Lv, P., & Xu, J. (2021). A novel strategy for D-psicose and lipase co-production using a co-culture system of engineered Bacillus subtilis and Escherichia coli and bioprocess analysis using metabolomics. Bioresources and Bioprocessing, 8, 77. https://doi.org/10.1186/s40643-021-00429-8
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Gullapalli, P., Takata, G., Poonperm, W., Rao, D., Morimoto, K., Akimitsu, K., Tajima, S., & Izumori, K. (2007). Bioproduction of D-psicose from allitol with Enterobacter aerogenes IK7: a new frontier in rare ketose production. Bioscience, Biotechnology, and Biochemistry, 71(12), 3048-3054. https://doi.org/10.1271/bbb.70450
Poonperm, W., Takata, G., Ando, Y., Sahachaisaree, V., Lumyong, P., Lumyong, S., & Izumori, K. (2007). Efficient conversion of allitol to D-psicose by Bacillus pallidus Y25. Journal of Bioscience and Bioengineering, 103(3), 282-285. https://doi.org/10.1263/jbb.103.282
Izumori, K. (2006). Izumoring: a strategy for bioproduction of all hexoses. Journal of Biotechnology, 124(4), 717-722. https://doi.org/10.1016/j.jbiotec.2006.04.016
Hassanin, H. A. M., Mu, W., Koko, M. Y. F., Zhang, T., Masamba, K., Jiang, B. (2017). Allitol: production, properties and applications. International Journal of Food Science and Technology, 52(1), 91-97. https://doi.org/10.1111/ijfs.13290
Takeshita, K., Shimonishi, T., & Izumori, K. (1996). Production of L-psicose from allitol by Gluconobacter frateurii IFO 3254. Journal of Fermentation and Bioengineering, 81(3), 212-215. https://doi.org/10.1016/0922-338X(96)82210-0
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Neuhauser, W., Steininger, M., Haltrich, D., Kulbe, K. D., & Nidetzky, B. (1998). A pH-Controlled Fed-Batch Process Can Overcome Inhibition by Formate in NADH-Dependent Enzymatic Reductions Using Formate Dehydrogenase-Catalyzed Coenzyme Regeneration. Biotechnology and Bioengineering, 60(3), 277-282. https://doi.org/10.1002/(SICI)1097-0290(19981105)60:3<277::AID-BIT2>3.0.CO;2-E
Kratzer, R., Woodley, J. M., & Nidetzky, B. (2015). Rules for biocatalyst and reaction engineering to implement effective, NAD(P)H-dependent, whole cell bioreductions. Biotechnology Advances, 33(8), 1641-1652. https://doi.org/10.1016/j.biotechadv.2015.08.006
Boldt, A., & Ansorge-Schumacher, M. B. (2020). Formate Dehydrogenase from Rhodococcus jostii (RjFDH) - A High-Performance Tool for NADH Regeneration. Advanced Synthesis und Catalysis, 362(19), 4109-4118. https://doi.org/10.1002/adsc.202000536
Tishkov, V. I., & Popov, V. O. (2004). Catalytic Mechanism and Application of Formate Dehydrogenase. Biochemistry (Moscow), 69(11), 1252-1267. https://doi.org/10.1007/s10541-005-0071-x
Xu, J., Zhou, H., Yu, H., Deng, T., Wang, Z., Zhang, H., Wu, J., & Yang, L. (2021). Computational design of highly stable and soluble alcohol dehydrogenase for NADPH regeneration. Bioresources and Bioprocessing, 8, 12. https://doi.org/10.1186/s40643-021-00362-w
Al-Rabiah, A. A., Boz, I., Akhmedov, V. M., Mostafa, M. M. M., & Bagabas, A. A. (2022). Highly Selective Gas-Phase Catalytic Hydrogenation of Acetone to Isopropyl Alcohol. Catalysts, 12(10), 1251. https://doi.org/10.3390/catal12101251
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Protein [Internet]. Bethesda (MD): National Library of Medicine (US), National Center for Biotechnology Information. Accession No. WP_265716617.1, SDR family oxidoreductase [Klebsiella pneumoniae]. Verfügbar unter: https://www.ncbi.nlm.nih.gov/protein/WP_265716617.1 (Zugriff am 22.12.2023)
Protein [Internet]. Bethesda (MD): National Library of Medicine (US), National Center for Biotechnology Information. Accession No. WP_063903495.1, SDR family oxidoreductase [Gluconobacter frateurii]. Verfügbar unter: https://www.ncbi.nlm.nih.gov/protein/WP_063903495.1/ (Zugriff am 22.12.2023)
Habenicht, A., Motejadded, H., Kiess, M., Wegerer, A., & Mattes, R. (1999). Xylose Utilisation: Cloning and Characterisation of the Xylitol Dehydrogenase from Galactocandida mastotermitis. Biological Chemistry, 380(12), 1405-1411. https://doi.org/10.1515/BC.1999.180
Protein [Internet]. Bethesda (MD): National Library of Medicine (US), National Center for Biotechnology Information. Accession No. WP_013084280.1, NAD(P)-dependent alcohol dehydrogenase [Priestia megaterium]. Verfügbar unter: https://www.ncbi.nlm.nih.gov/protein/WP_013084280.1/ (Zugriff am 22.12.2023)
Protein [Internet]. Bethesda (MD): National Library of Medicine (US), National Center for Biotechnology Information. Accession No. WP_070401870.1, SDR family oxidoreductase [Kozakia baliensis]. Verfügbar unter: https://www.ncbi.nlm.nih.gov/protein/WP_070401870.1/ (Zugriff am 22.12.2023)
Protein [Internet]. Bethesda (MD): National Library of Medicine (US), National Center for Biotechnology Information. Accession No. WP_068907433.1, SDR family oxidoreductase [Providencia heimbachae]. Verfügbar unter: https://www.ncbi.nlm.nih.gov/protein/WP_068907433.1/ (Zugriff am 22.12.2023)
V. L. Louis, L. Despons, A. Friedrich, et al. (2012). Pichia sorbitophila, an Interspecies Yeast Hybrid, Reveals Early Steps of Genome Resolution After Polyploidization. G3 Genes I Genomes I Genetics, 2(2), 299-311. https://doi.org/10.1534/g3.111.000745
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39
Protein [Internet]. Bethesda (MD): National Library of Medicine (US), National Center for Biotechnology Information. Accession No. MDQ0804260.1, Glucose 1-dehydrogenase [Priestia megaterium]. Verfügbar unter: https://www.ncbi.nlm.nih.gov/protein/MDQ0804260.1 (Zugriff am 22.12.2023)

## Patentansprüche

1. Verfahren zur Herstellung einer wässerigen Lösung enthaltend D-Psicose, indem aus D-Fructose, welche in einer wässerigen Lösung gelöst vorliegt, durch Behandeln mit einer Epimerase *in vitro* eine erste D-Psicose gebildet wird, wonach die erste D-Psicose durch Behandeln mit einer NAD(P)H-abhängigen Oxidoreduktase *in vitro* zu Allitol reduziert und nach Deaktivierung und/oder Ultrafiltration der Epimerase zur Bildung von D-Psicose mit einer entsprechenden NAD(P)⁺-abhängigen Oxidoreduktase behandelt wird, wonach die deaktivierte Epimerase und die Oxidoreduktasen entfernt werden, wobei die NAD(P)⁺-abhängige Oxidoreduktase zur Bildung von D-Psicose aus Allitol eine Aminosäuresequenz umfasst, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet.

2. Verfahren nach Anspruch 1, **dadurch gekennzeichnet, dass** der durch die Reduktion entstandene oxidierte Kofaktor NAD(P)⁺ mittels einer Alkoholdehydrogenase und einem sekundären Alkohol unter Bildung eines Ketons reduziert wird.

3. Verfahren nach Anspruch 2, **dadurch gekennzeichnet, dass** der sekundäre Alkohol 2-Propanol ist.

4. Verfahren nach einem der Ansprüche 1 bis 3, **dadurch gekennzeichnet, dass** es als Eintopf-Reaktion ohne Isolierung von Zwischenprodukten durchgeführt wird.

5. Verfahren nach einem der Ansprüche 1 bis 4, **dadurch gekennzeichnet, dass** die Enzyme als Lysat der entsprechenden, sie bildenden Zellen vorliegen.

6. Verfahren nach einem der Ansprüche 1 bis 5, **dadurch gekennzeichnet, dass** die NAD(P)H-abhängige Oxidoreduktase zur Reduktion der ersten D-Psicose zu Allitol eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

7. Verfahren nach einem der Ansprüche 2 bis 6, **dadurch gekennzeichnet, dass** die Alkoholdehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 18 von mindestens 80% aufweist,
ii) einer Aminosäuresequenz, die durch eine Nukleinsäure kodiert wird, die eine Identität zu SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 17 bindet.

8. Verfahren nach einem der Ansprüche 1 bis 7, **dadurch gekennzeichnet, dass** der durch die Reduktion entstandene oxidierte Kofaktor NAD(P)⁺ mittels einer Glucose-Dehydrogenase und D-Glucose unter Bildung von D-Gluconat reduziert wird.

9. Verfahren nach Anspruch 8, **dadurch gekennzeichnet, dass** die Glucose-Dehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 20 von mindestens 80% aufweist,
ii) einer Aminosäuresequenz, die durch eine Nukleinsäure kodiert wird, die eine Identität zu SEQ ID Nr. 19 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 19 bindet.

10. Verwendung einer Oxidoreduktase zur Bildung von D-Psicose aus Allitol, **dadurch gekennzeichnet, dass** die Oxidoreduktase eine Aminosäuresequenz umfasst, welche ausgewählt ist aus der Gruppe bestehend aus:
i) einer Aminosäuresequenz, die eine Identität zu SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3, SEQ ID Nr. 5, SEQ ID Nr. 7, SEQ ID Nr. 9 oder SEQ ID Nr. 11 bindet.

11. Verwendung einer Alkoholdehydrogenase zur Kofaktor-Regeneration, wobei die Alkoholdehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 18 von mindestens 80% aufweist,
ii) einer Aminosäuresequenz, die durch eine Nukleinsäure kodiert wird, die eine Identität zu SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 17 bindet.

12. Verwendung einer Glucose-Dehydrogenase zur Kofaktor-Regeneration, wobei die Glucose-Dehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 20 von mindestens 80% aufweist,
ii) einer Aminosäuresequenz, die durch eine Nukleinsäure kodiert wird, die eine Identität zu SEQ ID Nr. 19 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 19 bindet.

13. Verwendung einer H₂O-bildenden NAD(P)H-Oxidase zur Kofaktor-Regeneration, welche eine Aminosäuresequenz umfasst oder daraus besteht, die ausgewählt ist aus der Gruppe bestehend aus:
i) einer Aminosäuresequenz, die eine Identität zu SEQ ID Nr. 16 oder SEQ ID Nr. 14 von mindestens 80% aufweist,
ii) einer Aminosäuresequenz, die durch eine Nukleinsäure kodiert wird, die eine Identität zu SEQ ID Nr. 15 oder SEQ ID Nr. 13 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 15 oder SEQ ID Nr. 13 bindet.
